Europäisches Patentamt

European Patent Office

Office européen des brevets

(19)

(11) Veröffentlichungsnummer: **0 334 199**
A2

(12) EUROPÄISCHE PATENTANMELDUNG

(21) Anmeldenummer: 89104655.9

(51) Int. Cl.⁴: **C07C 143/12 , C07C 139/00**

(22) Anmeldetag: 16.03.89

(30) Priorität: 23.03.88 DE 3809822

(43) Veröffentlichungstag der Anmeldung:
27.09.89 Patentblatt 89/39

(84) Benannte Vertragsstaaten:
AT BE CH DE ES FR GB GR IT LI NL SE

(71) Anmelder: Henkel Kommanditgesellschaft auf Aktien
Postfach 1100 Henkelstrasse 67
D-4000 Düsseldorf 1(DE)

(72) Erfinder: Fabry, Bernd, Dr.
Danziger Strasse 31
D-4052 Korschenbroich(DE)
Erfinder: Piorr, Robert, Dr.
Kieselei 12
D-4030 Ratingen-Hösel(DE)

(54) Sulfonate von Estern ungesättigter Fettsäuren mit ungesättigten Fettalkoholen und Verfahren zur ihrer Herstellung.

(57) Fettsäurefettalkoholester-sulfonate, erhältlich durch Umsetzung von Fettsäurefettalkoholestern der Formel I
$R^1 - CO - OR^2$ (I)
in der die Gruppe $R^1 - CO$ den Rest einer ungesättigten $C_{16}$-$C_{22}$-Fettsäure und $R^2O$ - den Rest eines ungesättigten $C_{16}$-$C_{22}$-Fettalkohols bedeuten, mit Schwefeltrioxid sowie anschließender Neutralisation und Hydrolyse mit wässrigen Basen sind Verbindungen mit interessanten oberflächenaktiven Eigenschaften.

EP 0 334 199 A2

## Sulfonate von Estern ungesättigter Fettsäuren mit ungesättigten Fettalkoholen und Verfahren zu ihrer Herstellung.

Die Erfindung betrifft Sulfonate von Estern ungesättigter Fettsäuren mit ungesättigten Fettalkoholen und ein Verfahren zu ihrer Herstellung. Die Verbindungen der Erfindung stellen neue Produkte mit interessanten oberflächenaktiven Eigenschaften dar.

Sulfonate von Niedrigalkyl-estern ungesättigter Fettsäuren sind oberflächenaktive Vebindungen, die z.B. gemäß der EP-A 0 130 753 aus niedrigen Alkylestern ungesättigter Fettsäuren durch Umsetzung mit Schwefeltrioxid und anschließender Neutralisation sowie Hydrolyse hergestellt werden können. Die Struktur der letztlich erhaltenen Sulfonate ist noch nicht restlos aufgeklärt; es ist zu vermuten, daß dabei Gemische erhalten werden, die unter anderem aus Alken- und Hydroxyalkansulfonaten bestehen, vgl. J. Falbe, Surfactants in Consumer Products, p. 72-73, (1987), Springer-Verlag Berlin.

Es wurde nun gefunden, daß sich neue Sulfonate mit interessanten oberflächenaktiven Eigenschaften durch Umsetzung von Fettsäurefettalkoholestern der allgemeinen Formel I

$$R^1 - CO - OR^2 \quad (I)$$

in der die Gruppe $R^1 - CO -$ den Acylrest einer geradkettigen ungesättigten $C_{16}$-$C_{22}$-Fettsäure und $R^2 O -$ den Rest eines geradkettigen, ungesättigten $C_{16}$-$C_{22}$-Fettalkohols bedeuten, mit Schwefeltrioxid, gegebenenfalls in inerten Lösemitteln, in einem Verhältnis von 0,5 bis 1,8 mol Schwefeltrioxid pro olefinischer Doppelbindung bei Temperaturen von 15 bis 80 °C und Neutralisation sowie Hydrolyse der Sulfonierungsprodukte mit Basen herstellen lassen.

Da die erfindungsgemäß eingesetzten Fettsäurefettalkoholester sowohl im Fettsäurerest als auch im Fettalkoholrest mindestens je eine olefinische Doppelbindung aufweisen, können sie bei der Herstellung der Fettsäurefettalkoholester-sulfonate der Erfindung theoretisch mit mindestens 2 mol Schwefeltrioxid (je 1 mol pro Doppelbindung) reagieren. Es ist jedoch auch möglich, nicht sämtliche olefinische Doppelbindungen der eingesetzten Fettsäurefettalkoholester umzusetzen, so daß man z.B. auch durchschnittlich mit 1 mol Schwefeltrioxid sulfonierte Fettsäurefettalkoholester erhalten kann.

Die Fettsäurekomponente geeigneter Fettsäurefettalkoholester der Formel I kann aus geradkettigen, ungesättigten $C_{16}$-$C_{22}$-Fettsäuren, insbesondere aus Palmitoleinsäure, Ölsäure, Petroselinsäure, Gadoleinsäure und Erucasäure bestehen, wie sie in der Form technischer Fettsäuregemische aus pflanzlichen oder tierischen Ölen und Fetten erhalten werden können, z.B. aus Rindertalg, Schweineschmalz, Rapsöl, Sojaöl, Sonnenblumenöl und dergleichen.

Die Fettalkoholkomponente geeigneter Fettsäurefettalkoholester der Formel I kann aus geradkettigen, ungesättigten $C_{16}$-$C_{22}$-Fettalkoholen bestehen, insbesondere aus den zu den vorgenannten Fettsäuren analogen Fettalkoholen. Derartige Fettalkohole sind, insbesondere in der Form von technischen Gemischen, nach den bekannten Verfahren zur Herstellung von ungesättigten Fettalkoholen aus denselben natürlichen Rohstoffen zugänglich, wie die weiter oben beschriebenen ungesättigten Fettsäuren. Bevorzugte Ausgangsmaterialien für die Herstellung der erfindungsgemäßen Fettsäurefettalkoholsulfonate sind Erucylerucat und insbesondere Oleyloleat, die jeweils aus den entsprechenden technischen Fettsäuren und Fettalkoholen hergestellt sind.

Die Sulfonierung von Fettsäurefettalkoholestern der allgemeinen Formel I kann in der für Fettsäureniedrigalkylester an sich bekannten Weise erfolgen, z.B. mit gasförmigem Schwefeltrioxid in hierfür geeigneten Reaktoren, insbesondere von Typ der Fallfilmreaktoren. Dabei wird das Schwefeltrioxid mit Luft oder Stickstoff verdünnt und vorzugsweise in Form eines Gasgemisches mit ca. 1 bis 10 Vol-% Schwefeltrioxid eingesetzt. Bevorzugt wird die Reaktion in Abwesenheit von Lösemitteln durchgeführt.

Bei der Herstellung der Fettsäurefettalkoholester-sulfonate der Erfindung erfolgt die Sulfonierung bevorzugt mit einem Verhältnis von 0,6 bis 1,5, insbesondere 1,0 bis 1,3, mol Schwefeltrioxid pro mol olefinischer Doppelbindung. Bevorzugt ist weiterhin eine Sulfonierungstemperatur von 40 bis 60 °C.

Das so erhaltene Sulfonierungsprodukt wird anschließend mit Basen neutralisiert und hydrolysiert. Als Basen kommen hierbei Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak und organische Basen wie Ethanolamin, Diethanolamin oder Triethanolamin in Betracht. Alkalimetall- und Erdalkalimetallhydroxide und Ammoniak kommen dabei vorzugsweise in der Form mehr oder weniger konzentrierter wäßriger Lösungen zum Einsatz.

Gemäß einer bevorzugten Ausführungsform sind die Fettsäurefettalkylester-sulfonate der Erfindung dadurch herstellbar, daß das Sulfonierungsprodukt neutralisiert und unter Zufuhr von wässrigen Basen zur Einhaltung eines pH-Wertes von mindestens 6, insbesondere 6 bis 8, hydrolysiert wird. Dadurch lassen sich Nebenreaktionen des Schwefeltrioxids, z.B. alpha-Sulfonierungen an dem Acylrest und Spaltungen der Esterbindung, auf ein Minimum reduzieren.

Bevorzugt erfolgt die Hydrolyse der neutralisierten Sulfonierungsprodukte bei Temperaturen von mindestens 70°C innerhalb von 15 bis 240 Minuten, wobei die Obergrenze der Hydrolysetemperatur durch den Siedepunkt des Wassers bestimmt ist. Man kann jedoch auch bei Temperaturen über 100°C unter Druck hydrolysieren.

Wenn die eingesetzten Fettsäurefettalkoholester, wie bei der Herstellung aus technischen Fettsäure/Fettalkohol-Gemischen durchaus möglich ist, Anteile an gesättigten Fettsäurefettalkoholestern enthalten, können diese in den Fettsäurefettalkoholester-sulfonaten der Erfindung verbleiben oder, falls erwünscht, in an sich bekannter Weise z.B. durch Phasentrennung abgetrennt werden. Entsprechende Verfahren sind an sich für Sulfonate von Niedrigalkylestern ungesättigter Fettsäuren bekannt,vgl. die bereits genannte EP-A 0 130 753.

Die Erfindung wird im folgenden anhand bevorzugter Ausführungsbeispiele näher erläutert.

Beispiel 1.

Oleyloleat-sulfonat.

In einem 1l-Sulfierreaktor mit Gaseinleitungsrohr und Mantelkühlung wurden 539 g (1 mol) Oleyloleat (handelsübliches Produkt; IZ = 98, VZ = 105) vorgelegt und bei 40 bis 45°C mit 160 g (2 mol) gasförmigem Schwefeltrioxid umgesetzt. Das Schwefeltrioxid wurde durch Erhitzen von 246 g Oleum ausgetrieben, mit Stickstoff auf eine Konzentration von 5 Vol-% verdünnt und innerhalb von 70 min in den Ölsäureester eingeleitet, wobei die Temperatur des Reaktionsgemisches durch Kühlung unter 60°C gehalten wurde.

Das saure Reaktionsgemisch wurde nach der Sulfonierung auf 10°C abgekühlt und portionsweise mit 5 Gew.-% einer 35 %-igen Wasserstoffperoxid-Lösung gebleicht, wobei die Temperatur der Mischung durch Kühlung ebenfalls unterhalb von 60°C gehalten wurde.

Anschließend wurde das gebleichte Produkt in einer Lösung von 80 g (2 mol) Natronlauge in 250 ml Wasser eingerührt und 4 h bei 95°C hydrolysiert, wobei sich das Produkt entmischte. Nach dem Abkühlen wurde die obere Phase (ca. 10 Gew.-%), die unsulfoniertes Produkt enthielt, abgetrennt; die das Ölsäureoleylester(di)-sulfonat enthaltende untere Phase (ca. 90 Gew.-%) wurde mit Mineralsäure auf einen pH von 7 eingestellt.

| Kenndaten des Produkts: | | |
|---|---|---|
| Aniontensid-Gehalt: | 28 | Gew.-% |
| | (0,50 mval/g) | |
| Unsulfierte Anteile: | 7 | Gew.-% |
| Sulfat (als Natriumsulfat): | 2 | Gew.-% |
| Wasser (nach Fischer): | 63 | Gew.-% |
| Gesamt-Schwefel: | 2,7 | Gew.-% |
| Klett-Farbzahl: | 160 | |
| Jodzahl: | 21 | |
| Molgewicht (Zahlenmittel): | 563 | |

Hier, wie in den folgenden Beispielen 2 bis 4, wurden der Aniontensid-Gehalt und die unsulfierten Anteile nach den DGF-Einheitsmethoden, Stuttgart 1950 - 1984, H-III-10 bzw. G-III-6b ermittelt. Die Bestimmung der Klett-Farbzahl erfolgte nach 30 Minuten Bleichung mit 5 Gew.-%iger Wasserstoffperoxidlösung. Die Messung erfolgte bei einer Konzentration von 5 Gew.-% Aniontensid, bei pH 7, unter Verwendung einer 1 cm-Rundküvette und eines Blaufilters (400 bis 465 nm).

Beispiel 2.

Oleyloleat-sulfonat.

Unter den im Beispiel 1 angegebenen Bedingungen wurden 539 g (1 mol) Oleyloleat mit 80 g (1 mol) Schwefeltrioxid umgesetzt.

| Kenndaten des Produktes: | | |
|---|---|---|
| Aniontensid-Gehalt: | 10 | Gew.-% |
| | (0,23 mval/g) | |
| Unsulfierte Anteile: | 4 | Gew.-% |
| Sulfat (als Natriumsulfat): | 2 | Gew.-% |
| Wasser (nach Fischer): | 84 | Gew.-% |
| Gesamt-Schwefel: | 1,1 | Gew.-% |
| Klett-Farbzahl: | 78 | |
| Jodzahl: | 47 | |
| Molgewicht (Zahlenmittel): | 441 | |

Beispiel 3.

Sulfonierung von Oleyloleat in einem Fallfilmreaktor.

Der Fallfilmreaktor bestand aus Glas und wies ein von einem Heiz- und Kühlmantel umgebenes Rohr mit einer Länge von 1100 mm und einem Innendurchmesser von 6 mm auf. Der Reaktor war am Kopf mit einer Aufgabevorrichtung für das Esterausgangsmaterial und einem Gaseinleitungsrohr versehen. Gasförmiges Schwefeltrioxid, erzeugt durch Erhitzen von Oleum, wurde in Form einer Mischung mit Stickstoff (5 Vol.-% Schwefeltrioxid) eingesetzt.

Das Oleyloleat-Gemisch wurde mit einer konstanten Geschwindigkeit von 10 g/min aufgegeben. Die Zufuhr des Schwefeltrioxid-Stickstoff-Gemisches wurde so eingestellt, daß das Molverhältnis von Oleyloleat zu Schwefeltrioxid 1:2 betrug (1 mol Schwefeltrioxid pro Doppelbindung). Das rohe Reaktionsgemisch wurde kontinuierlich mit Wasserstoffperoxid gebleicht und mit Natronlauge neutralisiert; anschließend wurde wie im Beispiel 1 beschrieben hydrolysiert und aufgearbeitet.

| Kenndaten des Produkts: | | |
|---|---|---|
| Aniontensid-Gehalt: | 52 | Gew.-% |
| | (0,92 mval/g) | |
| Unsulfierte Anteile: | 11 | Gew.-% |
| Sulfat (als Natriumsulfat): | 4 | Gew.-% |
| Wasser (nach Fischer): | 33 | Gew.-% |
| Klett-Farbzahl: | 141 | |
| Jodzahl: | 18 | |
| Molgewicht (Zahlenmittel): | 563 | |

Beispiel 4.

Sulfonierung von Oleyloleat.

In der in Beispiel 1 beschriebenen Weise wurden 539 g (1 mol) Oleyloleat mit 192 g (2,4 mol) Schwefeltrioxid, entsprechend einem Molverhältnis von 1,2 mol Schwefeltrioxid pro olefinischer Doppelbindung umgesetzt; die weitere Aufarbeitung erfolgte ebenfalls wie im Beispiel 1.

EP 0 334 199 A2

| Kenndaten des Produkts: | | |
|---|---|---|
| Aniontensid-Gehalt: | 29 | Gew.-% |
| | (0,51 mval/g) | |
| Unsulfierte Anteile: | 6 | Gew.-% |
| Sulfat (als Natriumsulfat): | 3 | Gew.-% |
| Wasser (nach Fischer): | 62 | Gew.-% |
| Klett-Farbzahl: | 198 | |
| Jodzahl: | 16 | |
| Molgewicht (Zahlenmittel): | 571 | |

**Ansprüche**

1. Fettsäurefettalkoholester-sulfonate, erhältlich durch Umsetzung von Fettsäurefettalkoholestern der allgemeinen Formel I

$R^1$ - CO - $OR^2$     (I)

in der die Gruppe $R^1$ - CO - den Acylrest einer geradkettigen, ungesättigten $C_{16}$-$C_{22}$-Fettsäure und $R^2O$ -den Rest eines geradkettigen, ungesättigten $C_{16}$-$C_{22}$-Fettalkohols bedeuten, mit Schwefeltrioxid, gegebenenfalls in inerten Lösemitteln, in einem Verhältnis von 0,5 bis 1,8 mol Schwefeltrioxid pro olefinischer Doppelbindung bei Temperaturen von 15 bis 80°C und Neutralisation sowie Hydrolyse der Sulfonierungsprodukte mit Basen.

2. Fettsäurefettalkoholester-sulfonate nach Anspruch 1, erhältlich durch Sulfonierung in Abwesenheit von Lösemitteln mit einem mit inerten Gasen verdünnten Schwefeltrioxidstrom.

3. Fettsäurefettalkoholester-sulfonate nach Anspruch 1 oder 2, erhältlich durch Sulfonierung mit einem Verhältnis von 0,6 bis 1,5, insbesondere 1,0 bis 1,3, mol Schwefeltrioxid pro mol olefinischer Doppelbindung.

4. Fettsäurefettalkoholester-sulfonate nach mindestens einem der Ansprüche 1 bis 3, erhältlich durch Sulfonierung bei einer Temperatur von 40 bis 60°C.

5. Fettsäurefettalkoholester-sulfonate nach mindestens einem der Ansprüche 1 bis 4, erhältlich durch Neutralisation und Hydrolyse der Sulfonierungsprodukte mit wässrigen Basen unter Einhaltung eines pH-Wertes von mindestens 6, insbesondere 6 bis 8.

6. Fettsäurefettalkoholester-sulfonate nach mindestens einem der Ansprüche 1 bis 5, erhältlich durch Hydrolyse der neutralisierten Sulfonierungsprodukte bei Temperaturen von mindestens 70°C innerhalb von 15 bis 240 Minuten.

7. Verfahren zur Herstellung von Fettsäurefettalkoholester-sulfonaten, gekennzeichnet durch Umsetzung von Fettsäurefettalkoholestern der allgemeinen Formel I

$R^1$ - CO - $OR^2$     (I)

in der die Gruppe $R^1$ - CO - den Acylrest einer geradkettigen, ungesättigten $C_{16}$-$C_{22}$-Fettsäure und $R^2O$ -den Rest eines geradkettigen, ungesättigten $C_{16}$-$C_{22}$-Fettalkohols bedeuten, mit Schwefeltrioxid, gegebenenfalls in inerten Lösemitteln, in einem Verhältnis von 0,5 bis 1,8 mol Schwefeltrioxid pro olefinischer Doppelbindung bei Temperaturen von 15 bis 80°C und Neutralisation sowie Hydrolyse der Sulfonierungsprodukte mit wässrigen Basen.

8. Verfahren nach Anspruch 7, gekennzeichnet durch Sulfonierung in Abwesenheit von Lösemitteln mit einem mit inerten Gasen verdünnten Schwefeltrioxidstrom.

9. Verfahren nach Anspruch 7 oder 8, gekennzeichnet durch Sulfonierung mit einem Verhältnis von 0,6 bis 1,5, insbesondere 1,0 bis 1,3, mol Schwefeltrioxid pro mol olefinischer Doppelbindung.

10. Verfahren nach mindestens einem der Ansprüche 7 bis 9, gekennzeichnet durch Sulfonierung bei einer Temperatur von 40 bis 60°C.

11. Verfahren nach mindestens einem der Ansprüche 7 bis 10, gekennzeichnet durch Neutralisation und Hydrolyse der Sulfonierungsprodukte mit wässrigen Basen unter Einhaltung eines pH-Wertes von mindestens 6, insbesondere 6 bis 8.

12. Verfahren nach mindestens einem der Ansprüche 7 bis 11, gekennzeichnet durch Hydrolyse der neutralisierten Sulfonierungsprodukte bei Temperaturen von mindestens 70°C innerhalb von 15 bis 240 Minuten.

5